# EUROPÄISCHE PATENTSCHRIFT

(11) **EP 0 555 261 B1**
(45) Veröffentlichungstag und Bekanntmachung des Hinweises auf die Patenterteilung: **04.07.2001**
(21) Anmeldenummer: 91918266.7
(22) Anmeldetag: 21.10.1991
(51) Int. Cl.: A61L 24/00

(54) **WERKSTOFF ALS AUSGANGSMATERIAL ZUR HERSTELLUNG VON KNOCHENZEMENT, VERFAHREN ZU SEINER HERSTELLUNG, UND VERFAHREN ZUR HERSTELLUNG VON KNOCHENZEMENT**
STARTING MATERIAL FOR THE PRODUCTION OF BONE CEMENT, PROCESS FOR PRODUCING THE SAME AND PROCESS FOR PRODUCING BONE CEMENT
MATERIAU DE BASE POUR LA PRODUCTION DE CIMENT OSSEUX, SON PROCEDE DE PRODUCTION ET PROCEDE DE PRODUCTION DE CIMENT OSSEUX

(30) Priorität: 19.10.1990 DE 4033343
(43) Veröffentlichungstag der Anmeldung: 18.08.1993
(73) Patentinhaber: DRAENERT, Klaus, Dr.med., D-81545 München (DE)
(72) Erfinder: DRAENERT, Klaus, Dr.med., D-81545 München (DE)
(86) Internationale Anmeldenummer: EP9102000
(87) Internationale Veröffentlichungsnummer: WO9206717

(56) Entgegenhaltungen:
- EP-A- 0 016 906
- EP-A- 0 369 034
- EP-A- 0 386 525
- WO-A-86/01725
- WO-A-87/04110

## Beschreibung

Die Erfindung betrifft einen Werkstoff als Ausgangsmaterial zur Herstellung von Knochenzement, ein Verfahren zu dessen Herstellung sowie ein Verfahren zur Herstellung von Knochenzement.

In der Chirurgie und Orthopädie des Bewegungsapparates ist der Ersatz eines Gelenkes durch ein künstliches Gelenk zu einem Routineeingriff geworden. Das üblichste Verfahren dabei ist die Verankerung von Metallprothesenkomponenten im Knochen mittels einer plastischen Masse, welche auch als Knochenzement bekannt ist. Die bekannten Knochenzemente sind kalt polymerisierende Kunststoffe auf Zweikomponentenbasis, bestehend aus einem Monomer und einer pulverförmigen polymeren Komponente in Form von Kugeln oder Granula. Alle im Handel befindlichen üblichen Knochenzemente sind auf der Basis von Polymethylmethacrylaten (PMMA) hergestellt. Knochenzemente der angesprochenen Art sind beispielsweise im EP-A-0 016 906 beschrieben. Die Versuche zum Mischen der beiden Komponenten dieser Knochenzemente haben gezeigt, daß einerseits die Homogenität und andererseits die Blasenfreiheit die Hauptprobleme der Mischtechnik derartiger Zweikomponentenkunststoffe sind. Dies gilt insbesondere auch beim Unterrühren von Antibiotika, das oft schwierig ist. Hinzu kommt, daß das geschüttete Pulver beim Mischen aufstäubt und die Luft kontaminiert. Das Pulver kann in die Atemwege eingeschleust werden und zwar, wie experimentelle Untersuchungen gezeigt haben, bis in die Höhe des Hauptbronchus. Falls das Mischen unter Vakuum stattfindet, wird das Polymerpulver außerdem von der Vakuumpumpe angesaugt. Die Pulverkomponente ist somit das wesentliche Hindernis für die Herstellung des Knochenzements innerhalb eines geschlossenen Systems, was andererseits aus arbeitsmedizinischer Sicht sicherlich begrüßenswert wäre. Versuche, das Pulver zu evakuieren, sind bisher insofern unvollständig, als es auf diese Weise nicht gelingt, einen ausreichenden Polymerkugeleinschluß in der Matrix des Knochenzements zu garantieren. Noch größere Probleme bereitet hierbei das Inkorporieren von irgendwelchen Füllerpartikeln, wie Röntgenkontrastmitteln oder osteoinduktiven Substanzen.

Der Erfindung liegt somit die Aufgabe zugrunde, die vorstehenden Probleme zu beseitigen und das Handling des Polymerpulvers bei der Herstellung von Knochenzement zu vereinfachen.

Diese Aufgabe wird durch die Erfindung gemäß den Patentansprüchen gelöst. Die Erfindung geht dabei von dem Grundgedanken aus, die pulverförmige Polymerkomponente, deren Partikel üblicherweise Kugelform aufweisen, vor dem Mischen in einen Festkörper in Form eines Konglomerats zu überführen. Die Polymerkomponente des Knochenzements wird dabei ähnlich wie Würfelzucker verfestigt. Die Porosität dieses Konglomerats kann gezielt eingestellt und standardisiert werden, und das Polymer kann so verfestigt werden, daß ein Evakuieren möglich ist. Unterschiedliche Porisitäten lassen sich gezielt durch Variation der Größe der Polymerpartikel, der Schüttdichte und der Art der Verbindung der Polymerpartikel erzielen. Erfindungsgemäß liegt somit die Polymerkomponente des Knochenzements als poröser Preßling aus dicht gepackten und jeweils miteinander vorzugsweise punktförmig in Verbindung stehenden Polymerkugeln vor, zwischen denen ein Porensystem ausgebildet ist.

Mit der Erfindung ist es möglich, Bedingungen zu schaffen, die bei der Herstellung des Knochenzements ein homogenes Gemisch reproduzierbar garantieren, und zwar unabhängig von dem Geschick oder den Fähigkeiten desjenigen, der das Gemisch ansetzt. Ein erfindungsgemäß stabilisierter Polymerverband ist auch gefahrlos zu evakuieren, und zwar vor der Verpackung oder aber auch nach dem Mischen bzw. Ansetzen des Knochenzementstz.

Die erfindungsgemäß verfestigte Polymerkomponente des Knochenzements kann einfach und sicher wie eine Patrone gehandhabt werden.

Aufnahmen im Rasterelektronenmikroskop lassen erkennen, daß erfindungsgemäß zwischen den Polymerpartikeln genügend Raum für einen stabilen Polymer/Matrix-Verbund verbleibt; damit sind die Voraussetzungen für ein geschlossenes System bei der Herstellung und gegebenenfalls der Applikation des Knochenzements durch den Chirurgen geschaffen.

Die verfestigte Polymerkomponente kann auf einfache Weise einer Vorevakuierung unterworfen werden. Die Anrührversuche mit erfindungsgemäßen Polymerpatronen lassen erkennen, daß das Monomer nach seinem Zusatz sehr schnell in die vorgegebenen kapillaren Hohlräume zwischen den miteinander verbundenen Polymerpartikeln eingesaugt wird. Vorzugsweise erfolgt die Mischung der Komponenten unter Anwendung von Vakuum durch einen Rührer, der mit Teflon® oder einem anderen geeigneten Material, an dem der Knochenzement nicht anhängt, beschichtet ist. Es hat sich auch gezeigt, daß die Verteilung des Polymerpulvers erfindungsgemäß wesentlich gleichmäßiger erfolgt als wenn das Pulver ohne Herstellung eines Pulverkonglomerats direkt in die Monomerflüssigkeit eingeschüttet wird, insbesondere auch bei Vergleich mit Knochenzementen, bei denen das Pulver vorgelegt und dem Pulverberg das Monomer aufgeschüttet wird.

Das Polymerkonglomerat kann zur Versiegelung in eine Folie oder Membran aus PMMA eingeschweißt werden. Das PMMA wird dann beim Eintauchen in das Monomer angelöst und das Monomer wird in das Hohlraumsystem des Konglomerats eingesaugt.

Vorteilhafterweise kann das Konglomerat aus den Polymerpartikeln in Form eines Kreiszylinders, beispielsweise in Tablettenform gepreßt werden. Dabei ist es möglich, durch eine spezielle Ausbildung der Preßform einen oder mehrere Kanäle, beispielsweise ein Kanalsystem, in der Tablette vorzugeben, über das beim anschließenden Monomerzusatz das Monomer alle Polymerpartikel innerhalb der Tablette möglichst rasch und nahezu zur selben Zeit erreichen kann. Beispielsweise können mehrere konzentrisch angeordnete und sich in Axialrichtung des Tablettenzylinders erstreckende Kanäle in der Tablette vorgesehen sein. Es ist aber auch jedes andere Kanalsystem möglich, das gewährleistet, daß beim Mischen das Monomer möglichst rasch mit allen Polymerpartikeln in Berührung kommt.

Erfindungsgemäß können alle üblichen Knochenzement-Polymerkomponenten verwendet werden. Besonders, bevorzugt werden Polymere auf der Basis eines Polyacrylates oder eines Polymethacrylates, eines Polymethylmethacrylates, eines Copolymers eines Acrylates und Methacrylates und oder eine Mischung hieraus verwendet.

Erfindungsgemäß ist es besonders einfach, der in Form eines Konglomerats vorliegenden Polymerkomponente noch zusätzlich Füllerpartikel, beispielsweise Röntgenkontrastmittel oder knocheninduzierende Partikel in homogener Verteilung einzuverleiben. Die Füllerpartikel können in Form von Kugeln, Granula oder Fäden vorliegen. Bevorzugte Größen der Kugeln sind zwischen 5 und 300 *µ*m, besonders bevorzugt 50 bis 300 *µ*m und der Fasern zwischen 0,5 und 5 mm, vorzugsweise nicht größer als 3,5 mm.

Der erfindungsgemäße und als Ausgangsmaterial zur Herstellung von Knochenzement dienende Werkstoff kann auf verschiedene Weise hergestellt werden; wichtig dabei ist, daß die Polymerpartikel zu einem porösen Festkörper miteinander verbunden werden, der auch beim Evakuieren stabil ist. Beispielsweise können die Polymerpartikel durch Wärme punktförmig miteinander verschweißt werden. Hierzu sind üblicherweise Temperaturen zwischen 70 und 120°C erforderlich, je nach verwendetem Ausgangsmaterial. Die Polymerpartikel können auch durch Druckbeaufschlagung oberflächlich miteinander verbunden werden. Es kann auch durch gleichzeitige Anwendung von Hitze und Druck ein Preßling hergestellt und verfestigt werden. Bevorzugte Drücke liegen hier zwischen 1 und 10 bar, vorzugsweise bei etwa 5 bar.

Es ist auch möglich, das Konglomerat durch chemische Behandlung zu erzeugen, wobei vorzugsweise die Polymerpartikel oder Granula in eine Form gebracht und mit einem Lösungsmittel, wie Acetondampf, durchströmt werden, vorzugsweise unter gleichzeitigem Evakuieren.

Der erfindungsgemäße Werkstoff in Form eines Konglomerats oder einer Patrone aus miteinander verschweißten oder durch Druck verpreßten Polymerpartikeln hat in der Medizintechnik noch einen weiteren Vorteil. In letzter Zeit werden vermehrt PMMA-Materialien, die als Knochenzemente zugelassen sind, im Spritzgußverfahren verwandt. Um bioverträgliche Implantatkörper herzustellen, ist es erforderlich, aus dem Pulvermaterial Granulate herzustellen, die durch die Schnecken in den Fördermaschinen der Spritzgußmaschinen besser und reproduzierbarer als Pulver mitlaufen. Diese Granulate wurden bisher durch Mahlen ausgehärteter Knochenzemente erzeugt. Erfindungsgemäß ist es nunmehr möglich, die verfestigten Polymerpatronen bzw. Konglomerate als ganzes in die Maschine zu geben, wo sie sich selbst granulieren. Es ist außerdem auch möglich, die Polymerpatronen oder Konglomerate vorab in einer Granuliermaschine in einfacher Weise zu Granulat zu verarbeiten, das für Spritzgußmaschinen geeignet ist. Hierbei ist es vorteilhaft, die Polymerpartikel bei der Herstellung des Werkstoffs bzw. Konglomerats besonders fest zu verschweißen; hierzu können höhere Temperaturen von vorzugsweise 120 bis 180°, besonders bevorzugt etwa 150° angewandt werden, gegebenenfalls unter gleichzeitiger Druckbeaufschlagung.

Die Erfindung wird nachstehend anhand der Figur und der Beispiele näher erläutert.

Die Figur zeigt im demontierten Zustand die Teile einer Preßform zur Herstellung des erfindungsgemäßen Werkstoffes in Form eines Preßlings, der als Ausgangsmaterial zur Herstellung von Knochenzement verwendet wird.

### Beispiel 1

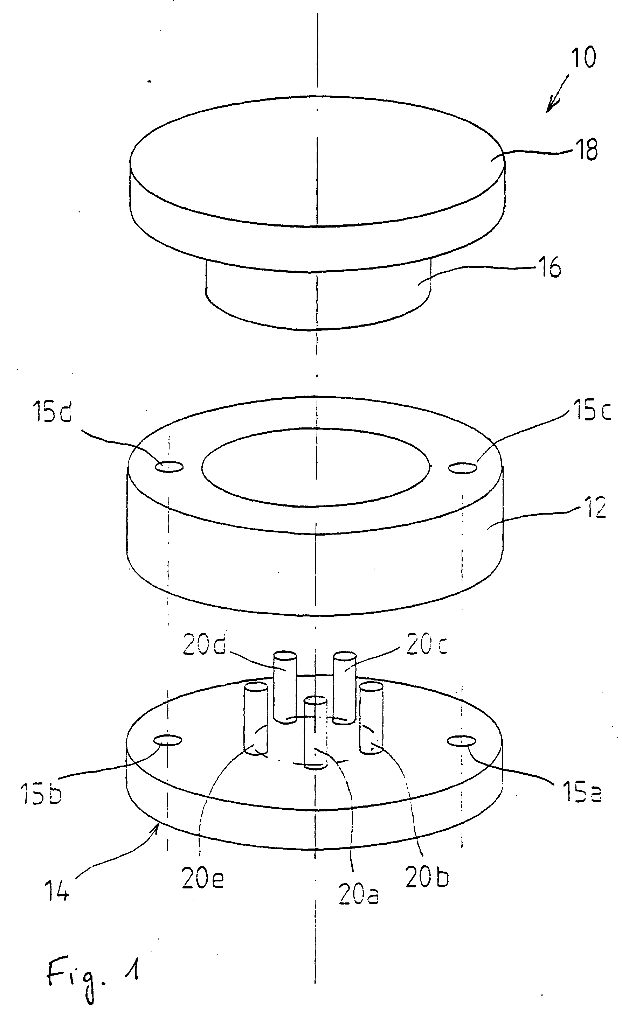

Gemäß Figur 1 ist als Preßform ein Zylinder 10, vorzugsweise aus Stahl, vorgesehen, der einen hohlzylindrischen Hauptteil 12 und einen abbehmbaren Boden 14 aufweist. Der Hauptteil 12 und der Boden 14 können beispielsweise mittels (nicht dargestellten) Schrauben miteinander verbunden werden, die durch die Bohrungen 15a bis 15d geführt werden. Ferner weist die Preßform einen zylindrischen Kolben 16 und einen hiermit einstückigen Deckel 18 auf. Der Außendurchmesser des Kolbens 16 entspricht im wesentlichen dem Innendurchmesser des hohlzylindrischen Hauptteils 12, und der Kolben 16 ist dicht in den Hauptteil 12 einführbar. In einer bevorzugten Ausführungsform weist der zylindrische Hauptteil 12 der Preßform einen Außendurchmesser von 90 mm, einen Innendurchmesser von 55 mm und eine Höhe von 20 mm auf. Am Boden 14 der Preßform sind fünf Zylinderstifte 20a bis 20e konzentrisch in einem Radius von 15 mm vom Zentrum des Bodens entfernt verteilt.

In einer bevorzugten Ausführungsform weisen die Stifte eine Höhe von 15 mm und einen Durchmesser von 6 mm auf.

Zur Herstellung des erfindungsgemäßen Werkstoffs in Form eines Preßlings werden zunächst die beiden Teile 12 und 14 der Preßform miteinander verbunden. Danach werden 40 g der Polymerkomponente eines handelsüblichen Knochenzementes in Form eines Perl- oder Kugelpolymerisats, dem 6 g Zirkondioxid als Röntgenkontrastmittel zugesetzt sind, eingeschüttet und mit Hilfe eines Rüttlers zum Setzen gebracht. Danach wird der Kolben 16 in den hohlzylindrischen Hauptteil 12 eingeführt und auf das Knochenzement-Perlpolymerisat aufgesetzt. Der Deckel 18 des Kolben 16 wird mit einer Kraft von etwa 100 N belastet, und die Preßform 30 Minuten lang in einem Trockenschrank bei 105°C belassen. Nach der Demontage der Preßform kann das auf diese Weise hergestellte Konglomerat aus den ursprünglich pulverförmigen Polymerpartikeln der Preßform als feste Tablette entnommen werden. Nach Zusatz von Dibenzoylperoxid, das beim Herstellen des Knochenzements als Polymerisationskatalysator dient, wird das tablettenförmige Konglomerat in einem geeigneten Tablettenrohr verpackt und für die Gassterilisation mittels Ethylenoxid vorbereitet.

### Beispiel 2

Das gemäß Beispiel 1 hergestellte Polymerpartikel-Konglomerat wird nach der Gassterilisation in einer sterilen Verpackungseinheit evakuiert und in einem Tablettenrohr vakuumverschweißt. Die Abmessungen des tablettenförmigen Konglomerats und des Tablettenrohrs werden derart gewählt, daß sie an ein Mischsystem adaptiert sind, das zur Herstellung des Knochenzements durch Mischen mit einem geeigneten Monomer verwendet wird. Ein derartiges Mischsystem ist beispielsweise in der EP-A-261182 beschrieben.

## Patentansprüche

1. Knochenzement-Polymerkomponente als Ausgangsmaterial zur Herstellung von Knochenzement, wobei die Polymerkomponente aus einem dreidimensionalen Konglomerat von Polymerpartikeln besteht , welches einen Festkörper darstellt und ein Porensystem einschliesst .

2. Knochenzement-Polymerkomponente nach Anspruch 1, wobei das Porensystem gleichförmig ausgebildet ist.

3. Knochenzement-Polymerkomponente nach Anspruch 1 1 oder 2 , wobei die Polymerpartikel aus Präpolymeren aus Polyacrylaten, Polymetacrylaten, Polymethylmethacrylaten, einem Copolymer eines Acrylates und eines Metacrylates und /oder einer Mischung derselben bestehen .

4. Knochenzement-Polymerkomponente nach einem der Ansprüche 1 bis 3 , wobei das Konglomerat evakuiert verpackt oder eingeschweisst ist , wobei das Konglomerat vorzugsweise in einem Behältnis evakuiert oder unter Vakuum eingeschweisst ist , wobei als Verpackungsmaterial vorzugsweise eine luftdicht PMMA-Folie verwendet wird .

5. Knochenzement-Polymerkomponente nach einem der Ansprüche 1-4 , wobei das Konglomerat zusätzlich Füllerpartikel in Form von Kugeln, Granula oder Fäden enthält .

6. Knochenzement-Polymerkomponente nach einem der Ansprüche 1-5 , wobei die Füllerpartikel Röntgenkontrastmittel oder Knochen-induzierende Partikel sind .

7. Knochenzement-Polymerkomponente nach einem der Ansprüche 5 oder 6 , wobei die Füllerpartikel eine Groesse zwischen 50 und 300 micron oder eine Faserlänge zwischen 0,5 und 5mm , vorzugsweise 3,5mm aufweisen .

8. Knochenzement-Polymerkomponente nach einem der Ansprüche 1-7 , wobei die Polymerpartikel zu einem Konglomerat in Tablettenform gepresst sind .

9. Knochenzement-Polymerkomponente nach einem der Ansprüche 1-8 , wobei in dem Konglomerat Hohlräume in Form eines Kanalsystemes vorgesehen sind .

10. Knchenzement-Polymerkomponente nach einem der Ansprüche 1-9 , wobei die Polymerpartikel in dem Konglomerat punktförmig verschweisst oder oberflächlich miteinander verbunden sind .

11. Verfahren zur Herstellung einer Knochenzement-Polymerkomponente nach einem der Ansprüche 1 bis 10 , wobei die anfänglich als Pulver vorliegenden Polymerpartikel der Polymerkomponente durch Temperaturen zwischen 70 und 120° Celsius miteinander punktförmig verschweisst und in ein Konglomerat überführt werden oder wobei die anfänglich als Pulver vorliegenden Polymerpartikel der Polymerkomponente durch Druckbeaufschlagung miteinander oberflächlich verbunden und in ein Konglomerat überführt werden oder wobei die anfänglich als Pulver vorliegende Polymerkomponente durch Anendung von Hitze zwischen 50 und 150° Celsius , vorzugsweise zwischen 70 und 100° Cels., unter gleichzeitiger Druchteaufschlagung zwischen 1 und 10 bar , vorzugsweise etwa 3 bis 7 bar , besonders bevorzugt etwa 5 bar , zu einem Konglomerat verfestigt wird oder wobei durch chemische Behandlung der anfänglich als Pulver vorliegenden Polymerpartikel ein Konglomerat erzeugt wird.

12. Verfahren zur Herstellung einer Knochenzement-Polymerkomponente nach Anspruch 11 , wobei das Pulver aus Polymerpartikeln unter Anwendung von Vakuum mit einem Lösungsmittel, vorzugsweise Aoetondämpfe, durchströmt wird, wodurch die Polymerpartikel miteinander verkleben und das Konglomerat bilden .

13. Verfahren zur Herstellung einer Knochenzement-Polymerkomponente nach Anspruch 11 , wobei für das Verfahren der Druckbeaufschlagung eine Pressform verwandt wird , die derart ausgebildet ist , dass in dem gebildeten Konglomerat ein Kanalsystem vorgegeben ist , wobei die Pressform vorzugsweise zylindrisch ist und in ihrem Inneren mehrere Einrichtungen , beispielsweise Stifte aufweist , die als Platzhalter für das Kanalsystem dienen.

14. Verwendung der Knochenzement-Polymerkomponente nach einem der Ansprüche 1-10 zur Herstellung von Knochenzement , wobei die Polymerkomponente durch Zusatz eines Polymerisationskatalysators und des Monomers zu einem selbsthärtenden Knochenzement angesetzt und gemischt wird.

15. Verfahren zur Herstellung eines Knochenzemenbes , welcher eine anfänglich aus Partikeln bestehende Polymerkomponente, einen Polymerisations Katalysator und das Monomer aufweist, wobei die Partikel der Polymerkomponente zunächst in ein Konglomerat in Form eines ein Porensystem aufweisenden Festkörpers überführt werden und anschliessend Polymerisations katalysator und Monomer zugesetzt und gemischt werden.

## Claims

1. A bone cement polymer component as base material for the production of bone cement, wherein said polymer component is made out of a three-dimensional conglomerate of polymer particles which represents a solid and includes a pore system.

2. The bone cement polymer component of claim 1, wherein said pore system is formed uniformly.

3. The bone cement polymer component of claims 1 or 2, wherein said polymer particles being composed of pre-polymers of polyacrylates, polymetacrylates, polymethylmetacrylates, a copolymer of acrylates and metacrylates, and/or a mixture thereof.

4. The bone cement polymer component of claims 1 to 3, wherein said conglomerate is individually packaged or welded, wherein said conglomerate is preferably evacuated in a receptacle or vacuum packaged by welding, wherein an air-tight polymethylmetacrylate foil is preferably used as packaging material.

5. The bone cement polymer component of claims 1 to 4, wherein said conglomerate additionally including filler particles in form of balls, granule or fibers.

6. The bone cement polymer component of claims 1 to 5, wherein said filler particle being a contrast medium for x-rays or bone inducing particles.

7. The bone cement polymer component of claims 5 to 6, wherein said filler particles including a length of between 50 and 300 micron or a fiber-length of between 0.5 to 5 mm, preferably 3.5 mm.

8. The bone cement polymer component of claims 1 to 7, wherein said polymer particles being pressed into a conglomerate in form of tablets.

9. The bone cement polymer component of claims 1 to 8, wherein hollow spaces in form of a canal system are provided in said conglomerate.

10. The bone cement polymer component of claims 1 to 9, wherein said polymer particles in the conglomerate are spot welded or connected superficially.

11. A method for producing a bone cement polymer component of claims 1 to 10, wherein said polymer particles of said polymer component initially being provided as powder will be spot welded together and transferred into a conglomerate, or wherein said polymer particles of said polymer component initially being provided as powder will be connected superficially by pressurization and transferred into a conglomerate, or wherein said polymer particles of said polymer component initially being provided as powder will through application of heat between 50 and 15° celsius, preferably between 70 and 100° celsius, with simultaneous pressurization of between 1 and 10 bar, preferably 3 to 7 bar, most preferably 5 bar, compacted, or wherein chemical treatment of said polymer particles initially being provided as powder will produce a conglomerate.

12. The method for producing a bone cement polymer component of claim 11, wherein said powder of polymer particles is flowed through by use of vacuum with a solvent, preferably acetone vapor, so that said polymer particles are bonded together and form said conglomerate.

13. The method for producing a bone cement polymer component of claim 11, wherein a mould is being used for the method of pressurization which is formed so that a canal system is provided in said formed conglomerate, wherein said mould is preferably cylindrical and includes several means inside, like pins which function as reservators for said canal system.

14. Use of said bone cement polymer component of claims 1 to 10 for producing bone cement, wherein said polymer component is prepared and mixed as self-setting bone cement by the addition of a polymerisation catalyst.

15. A method for producing bone cement, which includes a polymer component initially including particles, a polymer catalyst and the monomer, wherein the particles of said polymer component will first be converted into a conglomerate in form of a solid including a pore system and then be added and mixed with the polymer catalyst and the monomer.

## Revendications

1. Cément osseux à composante de polymères en tant que matériau de départ pour la fabrication de cément osseux, la composante de polymères étant constituée par un conglomérat de particules de polymères à trois dimensions représentant un corps solide et renfermant un système poreux.

2. Cément osseux à composante de polymères selon revendication 1, le système poreux étant réalisé selon la même forme.

3. Cément osseux à composante de polymères selon revendication 1 ou 2, les particules de polymères étant composées de prépolymères en polyacrylates, de polymétacrylates, de polymétacrylates de méthyle, d'un copolymère d'un acrylate et d'un métacrylate et/ou d'un mélange de ceux-ci.

4. Cément osseux à composante de polymères selon l'une des revendications 1 à 3, le conglomérat étant emballé sous vide ou enfermé par soudure, le conglomérat étant de préférence mis sous vide dans un récipient ou soudé sous vide, le matériau d'emballage utilisé étant de préférence une feuille en matière plastique au polymétacrylate de méthyle.

5. Cément osseux à composante de polymères selon l'une des revendications 1-4, le conglomérat renfermant en complément des particules de remplissage en forme de billes, de granulés ou de fils.

6. Cément osseux à composante de polymères selon l'une des revendications 1-5, les particules de remplissage étant constituées par du produit de contraste ou par des particules induisant la formation d'os.

7. Cément osseux à composante de polymères selon l'une des revendications 5 ou 6, les particules de remplissage étant d'une grosseur allant de 50 à 300 microns ou de fibres d'une longueur allant de 0,5 à 5 mm, de préférence de 3,5 mm.

8. Cément osseux à composante de polymères selon l'une des revendications 1-7, les particules de polymères étant pressées pour former un conglomérat en forme de pastilles.

9. Cément osseux à composante de polymères selon l'une des revendications 1-8, des espaces vides formant un système de canaux étant prévus dans le conglomérat.

10. Cément osseux à composante de polymères selon l'une des revendications 1-9, les particules de polymères étant soudées ponctuellement à l'intérieur du conglomérat ou reliées entre elles à la surface.

11. Procédé servant à la fabrication de cément osseux à composante de polymères selon l'une des revendications 1 à 10, les particules de polymères de la composante de polymères se trouvant initialement sous forme de poudre étant soudées entre elles sous l'effet de températures allant de 70 à 120° Celsius et transformées en un conglomérat, ou bien les particules de polymères de la composante de polymères se trouvant initialement sous forme de poudre étant reliées entre elles à la surface par application d'une pression et transformées en un conglomérat, ou bien les particules de polymères de la composante de polymères se trouvant initialement sous forme de poudre étant solidifiées pour former un conglomérat sous l'effet d'une chaleur allant de 50 à 150° Celsius, de préférence entre 70 et 100° Celsius, avec application simultanée d'une pression allant de 1 à 10 bars, de préférence de 3 à 7 bars, et plus particulièrement de préférence quelque 5 bars, ou encore réalisation d'un conglomérat par un traitement chimique des particules de polymères de la composante de polymères se trouvant initialement sous forme de poudre.

12. Procédé servant à la fabrication de cément osseux à composante de polymères selon revendication 11, la poudre de particules de polymères étant utilisée sous vide et traversée par un solvant, de préférence des vapeurs d'acétone, ce qui fait adhérer les particules de polymères les unes aux autres et réalise le conglomérat.

13. Procédé servant à la fabrication de cément osseux à composante de polymères selon revendication 11, un moule de pressage étant employé avec le procédé d'application d'une pression, réalisé de sorte à ce que dans le conglomérat formé un système de canaux soit préétabli, le moule de pressage étant de préférence cylindrique et présentant à l'intérieur plusieurs éléments, par exemples des broches qui servent à maintenir en place le système de canaux.

14. Utilisation de cément osseux à composante de polymères selon revendications 1-10 pour fabrication de cément osseux, la composante de polymères étant préparée en ajoutant un catalyseur de polymérisation et du monomère pour former un cément osseux qui durcit de lui-même, puis mélangée.

15. Procédé servant à la fabrication d'un cément osseux présentant initialement une composante de polymères constituée de particules, un catalyseur de polymérisation et le monomère, les particules de la composante de polymères étant tout d'abord transformées en un conglomérat en forme de corps solide à système poreux, le catalyseur de polymères et le monomère étant ensuite ajoutés et mélangés.
